# EUROPEAN PATENT APPLICATION

(11) **EP 4 414 379 A1**
(43) Date of publication of application: **14.08.2024**
(21) Application number: 22878525.9
(22) Date of filing: 04.10.2022
(51) Int. Cl.: C07K 14/47, A61K 38/17, A61P 3/06, A61P 3/10, A61P 9/00, A61P 9/10, A61P 9/12, C12P 21/02

(54) **CHOLESTEROL-FREE HIGH-DENSITY LIPOPROTEIN PARTICLES**

(30) Priority: 04.10.2021 JP 2021163384
(71) Applicant: PhoenixBio Co., Ltd., Hiroshima 739-0046 (JP)
(72) Inventor: KAKUNI, Masakazu, Hiroshima-shi, Hiroshima 739-0046 (JP); OKADA, Taro, Hiroshima-shi, Hiroshima 739-0046 (JP); YOSHIKAWA, Nami, Hiroshima-shi, Hiroshima 739-0046 (JP); TAKAHASHI, Masaki, Hiroshima-shi, Hiroshima 739-0046 (JP)
(74) Representative: J A Kemp LLP
(86) International application number: PCT/JP2022/037152
(87) International publication number: WO 2023/058657

(57) **Abstract**

A high density lipoprotein particle that does not contain, at least, cholesterol, wherein the high density lipoprotein particle is collected from a culture of hepatocytes; and a method for producing a high density lipoprotein particle that does not contain, at least, cholesterol, the method being characterized in that it comprises culturing hepatocytes, and then collecting the high density lipoprotein particle from the obtained culture.

## Description

### Technical Field

The present invention relates to a high density lipoprotein particle that does not contain cholesterol. The present particle has cholesterol uptake capacity and is utilized as a cholesterol transporter.

### Background Art

It has been reported that high density lipoprotein (HDL) is deeply associated with suppression of atherosclerosis (Non Patent Literatures 1 to 3). For this reason, the development of therapeutic drugs for the purpose of controlling the amount of HDL in blood is progressing, but it has been reported that there are cases where satisfactory medicinal effects cannot be obtained in clinical trials (Non Patent Literatures 4 to 7). In recent years, it has been revealed that the ability of HDL to pull out cholesterol from peripheral cells, that is the so-called "Cholesterol Uptake Capacity (CUC)," varies depending on the type of HDL (Non Patent Literatures 8). From this fact, it has been increasingly recognized that increasing HDL with high CUC is an important point required for therapeutic drugs.

HDL is mainly secreted into the blood after being newly synthesized in the liver, and pulls out free cholesterol from peripheral tissues via a transporter ABCA1 and intakes the free cholesterol. Free cholesterol in HDL is esterified by lecithin-cholesterol acyltransferase, so that it is converted to a cholesterol ester. This cholesterol ester is exchanged with neutral fats contained in VLDL and LDL, and HDL is matured. The mature HDL is taken up via an HDL receptor in the liver, and is then regenerated.

Thus, focusing on lipid metabolism such as CUC in HDL is urgently needed for the development of the above-mentioned therapeutic drugs, and furthermore, it is expected that it will greatly contribute to the overall understanding of the lipid metabolic function of hepatocytes (in particular, human hepatocytes), which are responsible for the synthesis, uptake and regeneration of HDL.

On the other hand, chimeric mice (PXB mice) in which 70% or more of the liver is replaced with human hepatocytes are known as cells having a lipid metabolic function equivalent to or similar to that of human hepatocytes (Non Patent Literature 9). In such PXB mice, since lipid transport is carried out between HDL and a low density lipoprotein (LDL) by Cholesterol Ester Transport protein secreted by human hepatocytes, the lipoprotein profile in the blood has been confirmed to have a configuration similar to that of human (Non Patent Literature 10).

In other words, it has been proved that hepatocytes separated from PXB mice (PXB cells) also have a lipid metabolic ability similar to that of fresh human hepatocytes (Non Patent Literature 11).

### Citation List

### Non Patent Literature

Non Patent Literature 1: Gordon T, Castelli WP, Hjortland MC, Kannel WB, Dawber TR. High density lipoprotein as a protective factor against coronary heart disease. The Framingham Study. Am J Med. 1977 May; 62(5): 707-14. doi: 10.1016/0002-9343(77) 90874-9. PMID: 193398.
Non Patent Literature 2: 1: Gordon DJ, Probstfield JL, Garrison RJ, Neaton JD, Castelli WP, Knoke JD, Jacobs DR Jr, Bangdiwala S, Tyroler HA. High-density lipoprotein cholesterol and cardiovascular disease. Four prospective American studies. Circulation. 1989 Jan; 79(1): 8-15. doi: 10.1161/01.cir.79.1.8. PMID: 2642759.
Non Patent Literature 3: Robins SJ, Collins D, Wittes JT, Papademetriou V, Deedwania PC, Schaefer EJ, McNamara JR, Kashyap ML, Hershman JM, Wexler LF, Rubins HB; VA-HIT Study Group. Veterans Affairs High-Density Lipoprotein Intervention Trial. Relation of gemfibrozil treatment and lipid levels with major coronary events: VA-HIT: a randomized controlled trial. JAMA. 2001 Mar 28; 285(12): 1585-91. doi: 10.1001/jama.285.12.1585. PMID: 11268266.
Non Patent Literature 4: Schwartz GG, Olsson AG, Abt M, Ballantyne CM, Barter PJ, Brumm J, Chaitman BR, Holme IM, Kallend D, Leiter LA, Leitersdorf E, McMurray JJ, Mundl H, Nicholls SJ, Shah PK, Tardif JC, Wright RS; dal-OUTCOMES Investigators. Effects of dalcetrapib in patients with a recent acute coronary syndrome. N Engl J Med. 2012 Nov 29; 367(22): 2089-99. doi: 10.1056/NEJMoa1206797. Epub 2012 Nov 5. PMID: 23126252.
Non Patent Literature 5: HPS2-THRIVE Collaborative Group, Landray MJ, Haynes R, Hopewell JC, Parish S, Aung T, Tomson J, Wallendszus K, Craig M, Jiang L, Collins R, Armitage J. Effects of extended-release niacin with laropiprant in high-risk patients. N Engl J Med. 2014 Jul 17; 371(3): 203-12. doi: 10.1056/NEJMoa1300955. PMID: 25014686.
Non Patent Literature 6: Barter PJ, Caulfield M, Eriksson M, Grundy SM, Kastelein JJ, Komajda M, Lopez-Sendon J, Mosca L, Tardif JC, Waters DD, Shear CL, Revkin JH, Buhr KA, Fisher MR, Tall AR, Brewer B; ILLUMINATE Investigators. Effects of torcetrapib in patients at high risk for coronary events. N Engl J Med. 2007 Nov 22; 357(21): 2109-22. doi: 10.1056/NEJMoa0706628. Epub 2007 Nov 5. PMID: 17984165.
Non Patent Literature 7: Schwartz GG, Olsson AG, Abt M, Ballantyne CM, Barter PJ, Brumm J, Chaitman BR, Holme IM, Kallend D, Leiter LA, Leitersdorf E, McMurray JJ, Mundl H, Nicholls SJ, Shah PK, Tardif JC, Wright RS; dal-OUTCOMES Investigators. Effects of dalcetrapib in patients with a recent acute coronary syndrome. N Engl J Med. 2012 Nov 29; 367(22): 2089-99. doi: 10.1056/NEJMoa1206797. Epub 2012 Nov 5. PMID: 23126252.
Non Patent Literature 8: Khera AV, Cuchel M, de la Llera-Moya M, Rodrigues A, Burke MF, Jafri K, French BC, Phillips JA, Mucksavage ML, Wilensky RL, Mohler ER, Rothblat GH, Rader DJ. Cholesterol efflux capacity, high-density lipoprotein function, and atherosclerosis. N Engl J Med. 2011 Jan 13; 364(2): 127-35. doi: 10.1056/NEJMoa1001689. PMID: 21226578; PMCID: PMC3030449.
Non Patent Literature 9: Tateno C, Kawase Y, Tobita Y, Hamamura S, Ohshita H, Yokomichi H, Sanada H, Kakuni M, Shiota A, Kojima Y, Ishida Y, Shitara H, Wada NA, Tateishi H, Sudoh M, Nagatsuka S, Jishage K, Kohara M. Generation of Novel Chimeric Mice with Humanized Livers by Using Hemizygous cDNA-uPA/SCID Mice. PLoS One. 2015 Nov 4; 10(11): e0142145. doi: 10.1371/journal.pone.0142145. PMID: 26536627; PMCID: PMC4633119.
Non Patent Literature 10: Papazyan R, Liu X, Liu J, Dong B, Plummer EM, Lewis RD 2nd, Roth JD, Young MA. FXR activation by obeticholic acid or nonsteroidal agonists induces a human-like lipoprotein cholesterol change in mice with humanized chimeric liver. J Lipid Res. 2018 Jun; 59(6): 982-993. doi: 10.1194/jlr.M081935. Epub 2018 Mar 20. PMID: 29559521; PMCID: PMC5983391.
Non Patent Literature 11: Hata K, Sayaka T, Takahashi M, Sasaki A, Umekawa Y, Miyashita K, Ogura K, Toshima G, Maeda M, Takahashi J, Kakuni M. Lipoprotein profile and lipid metabolism of PXB-cells, human primary hepatocytes from liver-humanized mice: proposal of novel in vitro system for screening anti-lipidemic drugs. Biomed Res. 2020; 41(1): 33-42. doi: 10.2220/biomedres.41.33. PMID: 32092738.

### Summary of Invention

### Technical Problem

In view of the above-described background, it has been desired to develop a particle that has high cholesterol uptake capacity and can be utilized as a cholesterol transporter.

As a result of intensive studies conducted directed towards achieving the aforementioned object, the present inventor has found that high density lipoprotein particles contained in a culture of fresh human hepatocytes or cells having a lipid metabolic function similar thereto (for example, PXB mouse-derived hepatocytes) have cholesterol uptake capacity, thereby completing the present invention.

### Solution to Problem

Specifically, the present invention is as follows.
[1] A high density lipoprotein particle that does not contain, at least, cholesterol, wherein the high density lipoprotein particle is collected from a culture of hepatocytes.
[2] The particle according to the above [1], wherein the hepatocytes are human hepatocytes, or non-human-derived hepatocytes having a lipid metabolic function equivalent to that of the human hepatocytes.
[3] The particle according to the above [2], wherein the non-human-derived hepatocytes are hepatocytes derived from a non-human animal in which at least 70% of the liver is replaced with human hepatocytes.
[4] The particle according to the above [1], which has cholesterol uptake capacity.
[5] The particle according to the above [3], wherein the non-human animal is a mouse.
[6] A cholesterol transporter comprising the particle according to any one of the above [1] to [5].
[7] The transporter according to the above [6], which pulls out cholesterol from tissues and transports it to the liver.
[8] The transporter according to the above [7], wherein the tissues are circulatory system tissues.
[9] The transporter according to the above [8], wherein the cholesterol in the circulatory system tissues is cholesterol in atherosclerotic plaques.
[10] A method for producing a high density lipoprotein particle that does not contain, at least, cholesterol, the method being characterized in that it comprises culturing hepatocytes, and then collecting the high density lipoprotein particle from the obtained culture.
[11] The method according to the above [10], wherein the hepatocytes are human hepatocytes, or non-human-derived hepatocytes having a lipid metabolic function equivalent to that of the human hepatocytes.
[12] The method according to the above [10], wherein the non-human-derived hepatocytes are hepatocytes derived from a non-human animal in which at least 70% of the liver is replaced with human hepatocytes.
[13] The method according to the above [10], wherein the high density lipoprotein particle has cholesterol uptake capacity.
[14] The method according to the above [12], wherein the non-human animal is a mouse.
[15] A pharmaceutical composition for circulatory system disease, comprising the particle according to any one of the above [1] to [5].
[16] A pharmaceutical composition for circulatory system disease, comprising the transporter according to the above [6].
[17] The pharmaceutical composition according to the above [15], wherein the circulatory system disease is at least one disease selected from the group consisting of hypercholesterolemia, familial hypercholesterolemia, atherosclerosis, peripheral vascular disease, dyslipidemia, angina pectoris, ischemia, stroke, myocardial infarction, hypertension, and vascular complications of diabetes.
[18] The pharmaceutical composition according to the above [16], wherein the circulatory system disease is at least one disease selected from the group consisting of hypercholesterolemia, familial hypercholesterolemia, atherosclerosis, peripheral vascular disease, dyslipidemia, angina pectoris, ischemia, stroke, myocardial infarction, hypertension, and vascular complications of diabetes.

### Advantageous Effects of Invention

According to the present invention, cholesterol-free HDL is provided. The HDL of the present invention is characterized in that it does not contain cholesterol, and since the HDL of the present invention has high ability to recover cholesterol, it is useful as a cholesterol transporter.

### Brief Description of Drawings

[Figure 1] Figure 1 is a schematic view of the HDL of the present invention.
[Figure 2] Figure 2 is a view showing the measurement results of apolipoproteins contained in the culture supernatant of PXB-cells.
[Figure 3] Figure 3 is a view showing the results obtained by examining the cholesterol uptake ability of HDL.
[Figure 4] Figure 4 is a view showing lipids contained in lipoprotein fractions.

### Description of Embodiments

### 1. Overview

The present invention relates to a high density lipoprotein particle that does not contain, at least, cholesterol. The particle of the present invention is collected from a culture of hepatocytes, and has cholesterol uptake capacity.

High density lipoprotein (HDL), one type of lipid particle, has a function of pulling out (collecting) cholesterol from tissues and organs in the body, and in recent years, the recovery of the cholesterol-collecting function of HDL is becoming a target of drug discovery. HDL contained in the blood of animals including humans always contains cholesterol, although there are some differences.

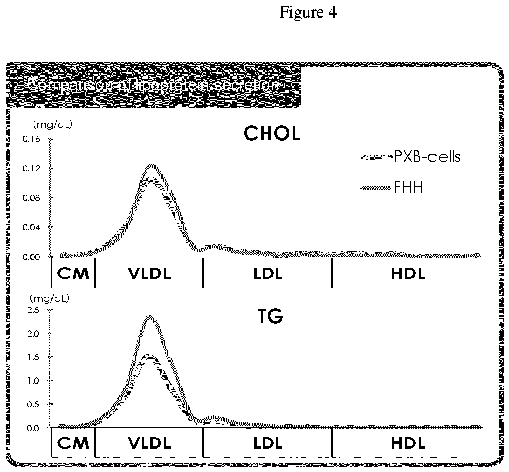

However, in the present invention, as a result that a culture of hepatocytes, in particular, a culture supernatant of hepatocytes was divided into fractions of a very low density lipoprotein (VLDL), a low density lipoprotein (LDL), and HDL, and then, the amount of cholesterol contained in each of the aforementioned fractions was confirmed, it was found that the HDL fraction contained almost no cholesterol (Figure 4). In Figure 4, the upper panel shows the cholesterol content, and the lower panel shows the neutral fat content. It was confirmed that there were almost no peaks in the HDL fraction for both cholesterol and neutral fats.

With regard to the fact that no cholesterol is contained in the HDL fraction in the above-described culture supernatant, whether HDL itself does not exist, or whether HDL exists but HDL does not have the function to absorb cholesterol, has been unknown.

Thus, in the present invention, the protein constituting HDL (APOA1) was semi-quantitatively quantified, and the cholesterol-collecting function of HDL was measured.

As a result, it could be confirmed that HDL capable of pulling out cholesterol exists in the above-described culture supernatant.

The present invention has been completed based on the above-described findings.

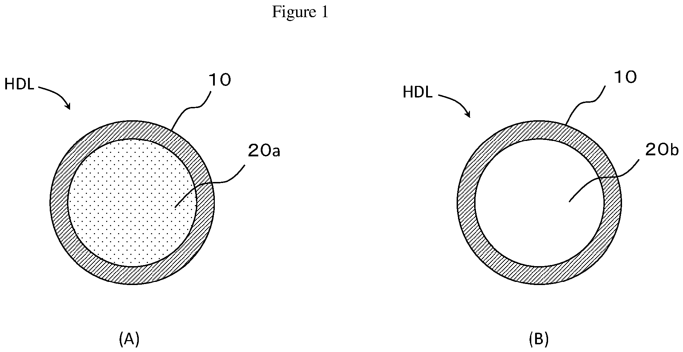

Figure 1 is a schematic view of the high density lipoprotein particle of the present invention (also referred to as "the particle of the present invention"). In Figure 1, panel A is a schematic view of normally existing HDL. HDL is a spherical particle as a whole, and forms two regions that are a surface layer portion 10 (outer shell) and an internal portion 20 (inner shell). The surface layer portion 10 of HDL is a membrane composed of apoproteins, phospholipids and free cholesterol, while an internal portion 20a thereof is mainly composed of cholesterol, fat-soluble vitamins and neutral fats. Panel B is a schematic view of the HDL particle obtained by the present invention.

In the particle of the present invention, the surface layer portion 10 is a membrane composed of apoproteins, phospholipids and free cholesterol, and an internal portion 20b thereof contains fat-soluble vitamins but does not contain, at least, cholesterol (cholesterol ester). In addition, in one aspect of the present invention, neither cholesterol nor neutral fats are contained. Herein, the term "not contain" or "...-free" that is used with respect to neutral fats or cholesterol means that the particle of the present invention does not contain at all cholesterol (the content percentage is zero), or does not substantially contain cholesterol. It means that the content of neutral fats in the present particle is 5% or less, preferably 1% or less, and the content of cholesterol in the present particle is 10% or less, preferably 7% or less.

### 2. Cells to be cultured

The cells used for the culture to obtain the particle of the present invention are human hepatocytes, or cells having a lipid metabolic function that is the same as or is similar to that of the human hepatocytes.

### (1) Human hepatocytes

Human hepatocytes (hepatic parenchymal cells) can be obtained from liver tissue harvested by biopsy or surgery. It is also possible to use established human hepatocyte lines.

Liver tissues harvested by biopsy or surgery are prepared as a cell dispersion by a conventional method such as a collagenase perfusion method, and the obtained dispersed cells are placed in a culture medium such as DMEM, and are then filtered through a gauze or a mesh. Since the obtained cell suspension contains Kupffer cells, astrocytes, bile duct epithelial cells, sinusoidal endothelial cells, liver stem cells and the like, as well as hepatic parenchymal cells, the cell suspension is subjected to centrifugation, so that fresh hepatocytes of interest can be obtained. For example, since the specific gravity of hepatic parenchymal cells is heavy, the human hepatocytes can be recovered by centrifugation at 50 x g for about 2 minutes.

The recovered fresh human hepatocytes are used for the culture described below.

### (2) Cells having lipid metabolic function equivalent to or simimar to that of human hepatocytes

As another aspect of the present invention, in addition to the above-described fresh human hepatocytes, cells having a lipid metabolic function equivalent to or simiolar to that of human hepatocytes (referred to herein as "equivalent hepatocytes") can be used. The term "equivalent to or similar to" means a lipid metabolic function that can produce the same value within at least 3-fold range, for example, within 3-fold range, within 2.5-fold range, within 2-fold range, or within 1.5-fold range, compared with the lipid metabolic function posessed by fresh human hepatocytes. For example, a system of measuring a lipoprotein is considered as a lipid metabolic function derived from human hepatocytes. When fresh human hepatocytes secrete x mg of lipoproteins, certain cells can be said to be equivalent hepatocytes if the certain cells secrete within 1/3 to 3 times of x mg.

Examples of such equivalent hepatocytes may include cells derived from non-human animals in which at least 70% of the liver has been replaced with human hepatocytes (hereafter referred to as "human hepatocyte chimeric animal"), and cells obtained by introducing a gene into somatic cells and allowing the somatic cells to differentiate into hepatocytes.

### (i) Cells derived from human hepatocyte chimeric animal

The cells used for the culture to obtain the particle of the present invention are non-human animal-derived hepatocytes, in which some or all of the hepatocytes in the liver have been replaced with human hepatocytes. In the present description, this non-human animal is referred to as a "human hepatocyte chimeric non-human animal."

The "non-human animal" is preferably a mammal, and is more preferably a rodent. Examples of the rodent may include a mouse, a rat, a guinea pig, a squirrel, and a hamster. Among others, a mouse or a rat, which is commonly used as a laboratory animal, is preferable.

The human hepatocyte chimeric non-human animal can be obtained by transplanting human hepatocytes into a liver disorder immunodeficient non-human animal according to a known method (e.g., JP Patent Publication (Kokai) No. 2002-45087 A).

The liver disorder immunodeficient non-human animal can typically be produced by inducing liver disorder to a genetically immunodeficient non-human animal or by inducing immunodeficiency to a genetically liver-disordered animal. Examples of such a genetically immunodeficient animal may include an SCID mouse, a NUDE mouse, a RAG2 knockout mouse, a NOD mice, and a NOG mice. As such a genetically liver-disordered animal, a transgenic animal, into which liver disorder-inducing protein genes (a uPA gene, a tPA gene, etc.) linked under the control of an enhancer and/or a promoter of a hepatocyte-specifically expressing protein are introduced, can be utilized.

In the present invention, "human hepatocytes " transplanted into the liver disorder immunodeficient non-human animal are not particularly limited, as long as they are human-derived hepatocytes. For example, human hepatocytes isolated from human liver tissues by a conventional method such as a collagenase perfusion method can be utilized. Human liver tissues may be derived from healthy individuals or from patients affected with diseases such as fatty liver or liver cancer, and liver tissues derived from healthy individuals are preferable. The isolated human hepatocytes can be directly used as they are, or can also be used by being further purified.

For transplantation of human hepatocytes into such a liver disorder immunodeficient non-human animal, human hepatocytes are transplated into the liver via the spleen of the non-human animal, or are directly transplanted into the liver through the portal vein.

The non-human animal obtained by the above-described method is an animal in which at least 70% of the liver has been replaced with human hepatocytes. As a mouse in which at least 70% of the liver is replaced with human hepatocytes, a commercially avaiable product ("PXB-mouse," PhoenixBio Co., Ltd.) can also be used.

The recovery of human hepatocytes from the chimeric non-human animal can be carried out according to a conventional method such as a collagenase perfusion method. The recovered human hepatocytes may be directly used as they are, or the recovered human hepatocytes may also be purified using a monoclonal antibody that specifically recognizes human hepatocytes or non-human animal hepatocytes.

In the present description, hepatocytes harvested from the above-described "PXB-mouse" are also referred to as "PXB-cells".

In the present invention, hepatocytes harvested from the above-described human hepatocyte chimeric animal are used in the culture described below.

### (ii) Cells that were allowed to differentiate into hepatocytes

In another aspect of the present invention, a gene is introduced into somatic cells, so that the somatic cells can be allowed to differentiate into hepatocytes.

Methods of allowing somatic cells to differentiate into hepatocytes by gene transfer have been publicly known, and a mehtod of using induced pluripotent stem cells (iPS cells), a direct reprogramming method comprising directly reprogramming from somatic cells into hepatocytes, etc. can be adopted. For example, 4 transcriptional factors, Oct4, Sox2, Klf4 and c-Myc, which are abundantly expressed in embryonic stem cells, are allowed to artificially express in somatic cells, so that the somatic cells can be reprogrammed and that induced pluripotent stem cells (iPS cells) can be obtained. After iPS cells have been obtained, a fibroblast growth factor (FGF), a bone morphogenetic protein (BMP), a hepatocyte growth factor (HGF), etc. are added alone or in combination thereof, as appropriate, into a medium, and the iPS cells are then cultured in the medium, so that the iPS cells can be allowed to differentiate into hepatocytes.

The hepatocytes obtained as described above are used in the culture described below.

In addition, a method of introducing two transcriptional factors (Hnf4α and Foxa1, Hnf4α and Foxa2, or Hnf4α and Foxa3) into somatic cells has been known as a direct reprogramming method.

### 3. Cell culture

The culture vessel used for cell culture is not particularly limited, as long as it is suitable for adherent culture. Examples of such a culture vessel may include a dish, a tissue culture dish, a multidish, a microplate, a microwell plate, a tube, a culture flask, and a culture bag.

In the present invention, human hepatocytes or equivalent hepatocytes can be cultured, using a medium that is commonly used for culturing animal cells. Examples of such a medium may include Dulbecco's modified Eagle's medium (DMEM), Williams medium E, and RPMI-1640 medium, and the preferred medium is DMEM. However, the medium used herein is not limited to these media.

Fetal bovine serum, a buffer, an antibiotic, a pH adjuster, etc. can be appropriately further added to the medium, as necessary.

The hepatocytes are seeded onto a culture vessel at a cell density of 2.1 × 10⁴ cells/cm² to 2.1 × 10⁶ cells/cm², and preferably 1.9 × 10⁵ cells/cm² to 2.3 × 10⁵ cells/cm². The hepatocytes can be cultured in a CO₂ incubator, under an atmosphere with a CO₂ concentration of 1% to 10%, preferably about 5%, at 30°C to 40°C, preferably at about 37°C.

The culture period of human hepatocytes or equivalent hepatocytes may be long enough for the cells to secrete and/or accumulate the particle of the present invention, and for example, such a culture period may be 6 days to 3 weeks, and preferably 2 weeks. Besides, the culture medium is be replaced partially or completely with a fresh medium every 1 to 4 days.

After completion of the culture, the particle of the present invention is harvested from the culture. The term "culture" refers to all of a culture supernatant, cultured cells, and a crushed material of the cultured cells.

In order to harvest the particle of the present invention, a centrifugation method, a precipitation method, a direct method, a gel filtration method, etc. can be employed.

### 4. Cholesterol-free HDL particle

### (1) Structure of HDL particle of the present invention

As described above, the HDL particle of the present invention has a structure, in which the surface layer portion is a membrane composed of apoproteins, phospholipids and free cholesterol, whereas the internal side contains fat-soluble vitamins, but does not contain, at least, cholesterol or does not contain both cholesterol and neutral fats.

Examples of the apolipoprotein constituting the HDL particle of the present invention may include apolipoprotein A1 (also referred to as "APOA1"; other apolipoproteins can be written down in the same manner), apolipoprotein A-II, apolipoprotein A-VI, apolipoprotein A-V, apolipoprotein C-I, apolipoprotein C-II, apolipoprotein C-III, apolipoprotein C-IV, apolipoprotein D, apolipoprotein E, apolipoprotein F, apolipoprotein H, apolipoprotein J, apolipoprotein L-1, and apolipoprotein M.

### (2) Confirmation that the HDL particle of the present invention is cholesterol-free

The confirmation (test method) that the HDL particle of the present invention is cholesterol-free can be carried out by gel filtration HPLC (LipoSEARCH), etc.

### (3) Cholesterol uptake ability

With regard to cholesterol uptake ability, labeled cholesterol is allowed to come into contact with HDL, and the HDL is then captured by magnetic beads on which an antibody for capturing HDL has been immobilized. Subequently, a complex of the magnetic beads, the HDL-capturing antibody and the HDL is allowed to react with a detection antibody that binds to the labeled cholesterol, and signals are detected.

The larger the amount of the signals, the more cholesterol that is incorporated into the HDL particle of the present invention.

Moreover, the HDL particle of the present invention is contained in the cell culture medium, and it is possible to evaluate its cholesterol uptake ability using the same procedure as those for HDL contained in animal serum. That is to say, after the cell culture medium has been allowed to react with polyethylene glycol 4000, APOB is removed by centrifugation, and HDL contained in the remaining supernatant solution fraction is mixed with the labeled cholesterol. Thereafter, the obtained mixture is incubated, so that the cholesterol is incorporated into the HDL. The method of capturing HDL and detecting labeled cholesterol is as described above.

### 5. Cholesterol transporter

The particle of the present invention can be utilized as a transporter that pulls out cholesterol from tissues and transports it to the liver. With regard to the particle size of the particle of the present invention, a particle, which has an average particle size of about 7.5 to 20 nm, preferably about 7.6 to 15 nm, can be used. The particle of the present invention having a particle size in the aforementioned range becomes a carrier suitable for movement in the capillaries of organs and tissues, while ensuring sufficient cholesterol loading.

The lower limit value of the average particle size is 7.5 nm or more, and the upper limit value is 20 nm or less. Accordingly, the range of average particle size is, for example, 7.5 nm to 18 nm, and preferably 7.6 nm to 15 nm.

Since the particle of the present invention is a substance constituting HDL, it can be safely used as a cholesterol delivery carrier. Therefore, in the medical field, the particle of the present invention can pulled out cholesterol from circulatory system tissues and other organs and tissues in the body, can accumulate it in the particle, and can transport it to the liver. In particular, in the circulatory system tissues, the particle of the present invention is excellent in terms of cholesterol efflux capacity from atherosclerotic plaques.

### 6. Pharmaceutical composition

The particle of the present invention can be further used as a pharmaceutical composition for circulatory system disease. The target circulatory system disease may be, for example, at least one disease selected from the group consisting of hypercholesterolemia, familial hypercholesterolemia, atherosclerosis, peripheral vascular disease, dyslipidemia, angina pectoris, ischemia, stroke, myocardial infarction, hypertension, and vascular complications of diabetes.

The administration form of the particle of the present invention used as a pharmaceutical composition includes parenteral and oral administration forms. Examples of the parenteral route of administration may include intravenous (IV) injection such as intravenous drip, intramuscular injection, intraperitoneal injection, and subcutaneous injection. Example of the oral route of administration may include a tablet, a capsule, a granule, a powder agent, and syrup. These forms are produced by known methods, and comprise a carrier, a diluent, and an excipient that are commonly used in the field of pharmaceutical formulation. For example, an injection is prepared by dissolving, suspending or emulsifying the particle of the present invention in a sterile aqueous or oily solution that is normally used for injections. As an aqueous solution for injections, a normal saline, an isotonic solution containing glucose and other auxiliary agents, or the like can be used, and the aqueous solution for injection can be used in combination with an appropriate dissolution aid such as, for example, alcohol, polyalcohol such as propylene glycol, and a nonionic surfactant.

The pharmaceutical composition comprising the particle of the present invention is administered to a patient with circulatory system disease in an amount sufficient to cure or, at least, to partially inhibit the symptoms of the disease. The administration method can be selected, as appropriate, depending on the age, symptoms, etc. of the patient. The effective dose of the pharmaceutical composition of the present invention is determined by using, as a guide, the fact that the serum total HDL particle count reaches 27.0 µmol/L or more, or that the serum concentration of small HDL or very small HDL that is defined by the particle size of 7.6 nm to 9.8 nm, etc., reaches 23.0 µmol/L or more. However, the pharmaceutical composition of the present invention is not limited to these doses. With regard to the administration period, the pharmaceutical composition of the present invention may be administered after the onset of the disease, or may also be administered prophylactically, when the onset of the disease is predicted in order to alleviate the symptoms at the time of onset.

### Examples

Hereinafter, the present invention will be more specifically described in the following Examples. However, these Examples are not intended to limit the scope of the present invention.

### 1. Culture of PXB-cells

Human hepatocytes were separated from PXB-mice produced by transplanting JFC (Bio reclamation IVT: Caucasian, 1Y, Male) into cDNA-uPAwild/+/SCID mice according to a collagenase perfusion method. The cell viability was 80% or more.

DMEM + 10% FBS were added to PXB-cells to prepare a cell suspension to achieve a cell density of 8 × 10⁵ cells/mL (2.1 × 10⁵ cells/cm²), and this cell suspension was added to a 24-well plates (BioCoat^{™} Collagen 124 well plate; Corning Japan) in an amount of 0.5 mL each per well. The plate, to which the cell suspension had been adde, was left at rest at room temperature for 15 to 30 minutes. After confirming that the cells lightly adhered to the bottom of the wells, the cells were cultured in an incubator (37°C, 5*%* CO₂). The starting day of culture was set as Day 0.

### < HDL-CUC measurement on Days 6 to 8 >

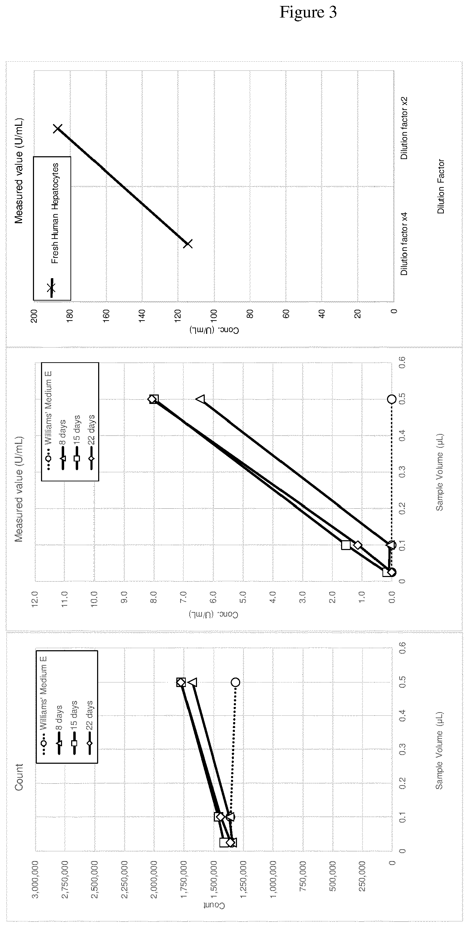

The medium was replaced with dHCGM at 0.5 mL each per well on Day 1 and Day 2. Thereafter, the medium was replaced with William's E + CM-4000 at 0.5 mL each per well on Day 6, and the culture supernatant was then recovered 2 days later, namely, on Day 8. The recovered supernatant sample was used in the analysis of cholesterol uptake ability by HDL (HDL-CUC) (corresponding to the "8 days" in Figure 3).

### < HDL-CUC measurement on Days 13 to 15 >

The medium was replaced with dHCGM at 0.5 mL each per well on Day 1, Day 2, Day 6, and Day 9. Thereafter, the medium was replaced with William's E + CM-4000 at 0.5 mL each per well on Day 13, and the culture supernatant was then recovered 2 days later, namely, on Day 15. The recovered supernatant sample was used in the analysis of cholesterol uptake ability by HDL (HDL-CUC) (corresponding to the "15 days" in Figure 3).

### < HDL-CUC measurement on Days 20 to 22 >

The medium was replaced with dHCGM at 0.5 mL each per well on Day 1, Day 2, Day 6, Day 9, Day 13, and Day 16. Thereafter, the medium was replaced with William's E + CM-4000 at 0.5 mL each per well on Day 20, and the culture supernatant was then recovered 2 days later, namely, on Day 22. The recovered supernatant sample was used in the analysis of cholesterol uptake ability by HDL (HDL-CUC) (corresponding to the "22 days" in Figure 3).

Besides, in order to obtain background values, the cholesterol uptake ability by HDL (HDL-CUC) with non-cultured William's E + CM-4000 was analyzed.

### < Apolipoprotein measurement on Days 13 to 15 >

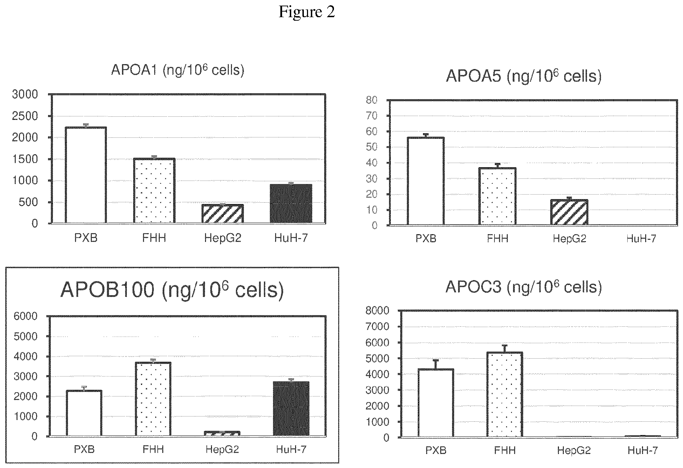

The medium was replaced with dHCGM at 1.0 mL each per well on Day 1. Then, the medium was replaced with dHCGM at 0.5 mL each per well on Day 2, Day 6, and Day 9. Thereafter, the medium was replaced with William's E + CM-4000 at 0.5 mL each per well on Day 13, and the culture supernatant was then recovered 2 days later, namely, on Day 15. The recovered supernatant sample was used in the measurement of apolipoproteins (corresponding to the "PXB" in Figure 2).

### 2. Culture of fresh human hepatocytes

Human non-frozen hepatocytes (HEP220-MW24; BIOPREDIC International) that had been inoculated on a 24-well plate, and a culture medium (Incubation Medium 100 mL; HIL214-100M; BIOPREDIC International) were obtained, and the cells were cultured in the culture medium for 1 day, while the date of acquisition was set at Day 0. The medium was replaced with William's E + CM-4000 at 0.5 mL each per well on Day 1, and the culture supernatant was then recovered 2 days later, namely, on Day 3. The recovered supernatant sample was used in the measurement of apolipoproteins (corresponding to the "FHH" in Figure 2) and the analysis of cholesterol uptake ability by HDL (HDL-CUC) (corresponding to the "Fresh human hepatocytes" in Figure 3).

### 3. Measurement of apolipoproteins

The apolipoproteins (APOA1, APOA5, APOB100, and APOC3) in the culture supernatant were quantified by using ELISA (enzyme-linked immunosorbent assay).

The results of quantification of the apolipoproteins in the culture supernatant by ELISA are shown in Figure 2.

Figure 2 is a view showing the results obtained by measuring the apolipoproteins contained in the culture supernatants of PXB-cells (corresponding to "PXB"), fresh human hepatocytes (corresponding to "FHH"), HepG2 (human hepatocarcinoma-derived cell line), and HuH-7 (human hepatocarcinoma-derived cell line). In the culture supernatant of the PXB-cells, APOA1, which constitutes HDL particles, is abundantly present. This indicates the presence of HDL particles containing APOA1 as a structural component in the culture supernatant.

For the purpose of removing APOB proteins from the culture supernatant for the measurement of cholesterol uptake capacity, a 22*%* PEG4000 solution was added to the specimen in an amount equal to that of the specimen, and the specimen was then pelleted by centrifugation. Since HDL that is composed of APOAI remains in the supernatant, the HDL function (cholesterol uptake capacity; HDL-CUC) was measured using this supernatant fraction. The process of a PEG treatment is as follows.

30 µL of specimen was dispensed into a 1.5-mL PROTEOSAVE tube, and 30 µL of 22*%* PEG4000 was then added thereto. The obtained mixture was left at rest at room temperature for 20 minutes, and was then centrifuged at 3000 rpm at 25°C for 15 minutes. Subsequently, the specimen was left at rest on ice for 10 minutes, and was then centrifuged at 10000 rpm at 4°C for 3 minutes. After completion of the centrifugation, the supernatants were recovered. The culture supernatant derived from the PXB-cells was diluted at dilution factors of 200-fold (corresponding to 0.025 µL of Sample Volume), 50-fold (corresponding to 0.1 µL of Sample Volume), and 10-fold (corresponding to 0.5 µL of Sample Volume), and the culture supernatant from the fresh human hepatocytes was diluted at dilution factors of 4-fold (corresponding to "Dilution factor × 4") and 2-fold (corresponding to "Dilution factor × 2"), followed by measuring.

The measurement was carried out using a fully automated high-sensitive immunoassay system for research use, HI-1000 (Sysmex). The procedure of adding reagents and the reaction time are as follows:
- R1 reagent (biotin-labeled cholesterol), 90 µL
- Specimen, 10 µL
- R2 reagent (anti-human APOAI antibody-labeled magnetic beads), 30 µL
- Reacted at 37°C for 369 seconds
- Washing (B/F separation)
- R3 reagent (streptavidin-ALP), 100 µL
- Reaction at 37°C for 568 seconds
- Wash (B/F separation)
- R4 reagent (luminescent substrate), 50 µL
- R5 reagent (buffer), 100 µL
- Reaction at 42°C for 305 seconds
- Luminescence measurement

The results are shown in Figure 3 and Figure 4. Figure 3 is a view showing the results obtained by examining HDL-CUC. Figure 4 is a view showing the lipids contained in lipoprotein fractions.

In the HDL-CUC test method, HDL particles incorporating biotin-labeled cholesterol therein are specifically selected with an anti-human APOAI antibody, and the biotin-labeled cholesterol incorporated into the HDL particles is then allowed to emit light, so that the cholesterol in HDL was quantified. Since a dilution factor-dependent increase in the luminescence was confirmed in the culture supernatants used in the test, it was demonstrated that HDL particles having a cholesterol-recovering function are present in the culture supernatants of the fresh human hepatocytes and the PXB-cells.

### Reference Signs List

10: Surface layer portion 20: Internal portion

## Claims

1. A high density lipoprotein particle that does not contain, at least, cholesterol, wherein the high density lipoprotein particle is collected from a culture of hepatocytes.

2. The particle according to claim 1, wherein the hepatocytes are human hepatocytes, or non-human-derived hepatocytes having a lipid metabolic function equivalent to that of the human hepatocytes.

3. The particle according to claim 2, wherein the non-human-derived hepatocytes are hepatocytes derived from a non-human animal in which at least 70% of the liver is replaced with human hepatocytes.

4. The particle according to claim 1, which has cholesterol uptake capacity.

5. The particle according to claim 3, wherein the non-human animal is a mouse.

6. A cholesterol transporter comprising the particle according to any one of claims 1 to 5.

7. The transporter according to claim 6, which pulls out cholesterol from tissues and transports it to the liver.

8. The transporter according to claim 7, wherein the tissues are circulatory system tissues.

9. The transporter according to claim 8, wherein the cholesterol in the circulatory system tissues is cholesterol in atherosclerotic plaques.

10. A method for producing a high density lipoprotein particle that does not contain, at least, cholesterol, the method being **characterized in that** it comprises culturing hepatocytes, and then collecting the high density lipoprotein particle from the obtained culture.

11. The method according to claim 10, wherein the hepatocytes are human hepatocytes, or non-human-derived hepatocytes having a lipid metabolic function equivalent to that of the human hepatocytes.

12. The method according to claim 10, wherein the non-human-derived hepatocytes are hepatocytes derived from a non-human animal in which at least 70% of the liver is replaced with human hepatocytes.

13. The method according to claim 10, wherein the high density lipoprotein particle has cholesterol uptake capacity.

14. The method according to claim 12, wherein the non-human animal is a mouse.

15. A pharmaceutical composition for circulatory system disease, comprising the particle according to any one of claims 1 to 5.

16. A pharmaceutical composition for circulatory system disease, comprising the transporter according to claim 6.

17. The pharmaceutical composition according to claim 15, wherein the circulatory system disease is at least one disease selected from the group consisting of hypercholesterolemia, familial hypercholesterolemia, atherosclerosis, peripheral vascular disease, dyslipidemia, angina pectoris, ischemia, stroke, myocardial infarction, hypertension, and vascular complications of diabetes.

18. The pharmaceutical composition according to claim 16, wherein the circulatory system disease is at least one disease selected from the group consisting of hypercholesterolemia, familial hypercholesterolemia, atherosclerosis, peripheral vascular disease, dyslipidemia, angina pectoris, ischemia, stroke, myocardial infarction, hypertension, and vascular complications of diabetes.
